# EUROPEAN PATENT APPLICATION

(11) **EP 0 955 307 A1**
(43) Date of publication of application: **10.11.1999**
(21) Application number: 99302110.4
(22) Date of filing: 18.03.1999
(51) Int. Cl.: C07H 17/08

(54) **Erythromycin a derivatives and methods for the preparation thereof**

(30) Priority: 14.04.1998 IL 12408498
(71) Applicant: Chemagis Ltd., Bnei Brak (IL)
(72) Inventor: Nadaka, Vladimir, Lod 71338 (IL); Kaspi, Joseph, Givataim 53201 (IL)
(74) Representative: Ablewhite, Alan James

(57) **Abstract**

The invention provides novel erythromycin A derivatives of the general formula I: 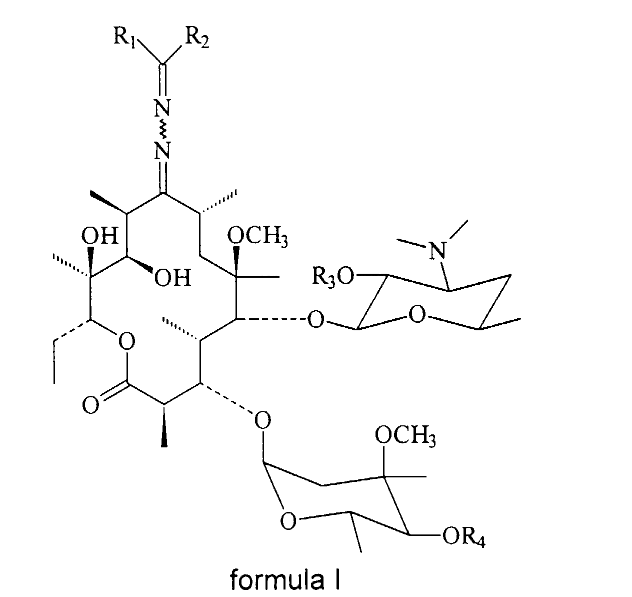 wherein R₁ is a hydrogen atom or an alkyl group having 1 to 6 carbon atoms; R₂ is an alkyl group having 1 to 6 carbon atoms or an unsubstituted or substituted aryl group; or R₁ and R₂ together form a linear or branched chain alkylene ring having 3 to 10 carbon atoms, an arylenealkylene group in which the alkylene moiety has 2 to 3 carbon atoms, or an arylenedialkylene group in which the alkylene moities have 1 to 2 carbon atoms; R₃ is a substituted silyl group; and R₄ is a hydrogen atom or substituted silyl group.

## Description

### Background of the Invention

### Field of the Invention

The present invention relates to novel erythromycin A derivatives and a method for their preparation, which novel derivatives are useful as intermediates for the preparation of the antibiotic drug 6-0-methylerythromycin A (clarithromycin).

### Description of Prior Art

Erythromycin A is regarded as one of the safest antibiotics. However, the compound decomposes rapidly under acidic conditions via intermolecular cyclization and loses its antibacterial activity. One approach to improve the stability is by methylation of the hydroxy group at the 6 position, thus preventing this cyclization. 6-O-methylerythromycin A shows an excellent effect for treatment of bacterial infections by oral administration, and therefore it is useful as an antibacterial agent.

There are several known methods for the preparation of 6-O-methylerythromycin A, for example, the methods for the selective methylation of the hydroxy group at the 6-position of erythromycin A derivatives which comprise converting erythromycin A derivatives having the protected hydroxy group at the 2'-position and the protected dimethylamino group at the 3'-position into the various kinds of the substituted oxime derivatives, followed by carrying out methylation of the hydroxy group at the 6-position, removal of the protecting groups, deoximation at the 9-position and regeneration of the dimethylamino group at the 3'-position to give 6-O-methylerythromycin A, which methods are described in European Patent 195,960, and European Patent 158,467. Similarly, an improved method for the selective methylation of the hydroxy group at the 6-position of erythromycin A 9-oxime derivatives is based on the protection of the hydroxy groups at 2'- and 4"- positions by the substituted silyl groups and the hydroxy group in 9-oxime moiety by an alkoxyalkyl group which are easily removed after the methylation of the hydroxy group at the 6-position is described in US Patent 4,990,602.

Although it is possible to methylate the 6-hydroxy group selectively by these methods, the methods described in said European Patents require complicated procedures such as catalytic reduction for removal of the protecting groups after methylation, and the method of said U.S. Patent requires purification by column chromatography.

### SUMMARY OF THE INVENTION

With this state of the art in mind, there is now provided according to the present invention a process for the selective methylation of the hydroxy group at the 6-position of an erythromycin A derivative based on the prior conversion of the erythromycin A derivative into a hydroxy protected erythromycin A 9-azine derivative and reacting the resulting erythromycin A 9-azine derivative with a methylating agent.

### DETAILED DESCRIPTION OF THE INVENTION

More specifically, the present invention provides novel erythromycin A derivatives represented by the general formula I: 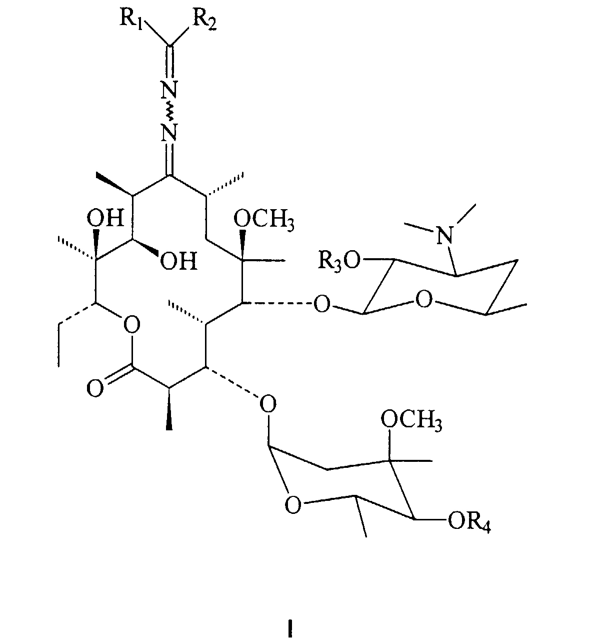 wherein R₁ is a hydrogen atom or an alkyl group having 1 to 6 carbon atoms; R₂ is an alkyl group having 1 to 6 carbon atoms or an unsubstituted or substituted aryl group; or R₁ and R₂ together form a linear or branched chain alkylene ring having 3 to 10 carbon atoms, an arylenealkylene group in which the alkylene moiety has 2 to 3 carbon atoms, or an arylenedialkylene group in which the alkylene moities have 1 to 2 carbon atoms; R₃ is a substituted silyl group; and R₄ is a hydrogen atom or substituted silyl group.

In especially preferred embodiments of the present invention there are provided 6-O-methylerythromycin A derivatives represented by the general formula II: 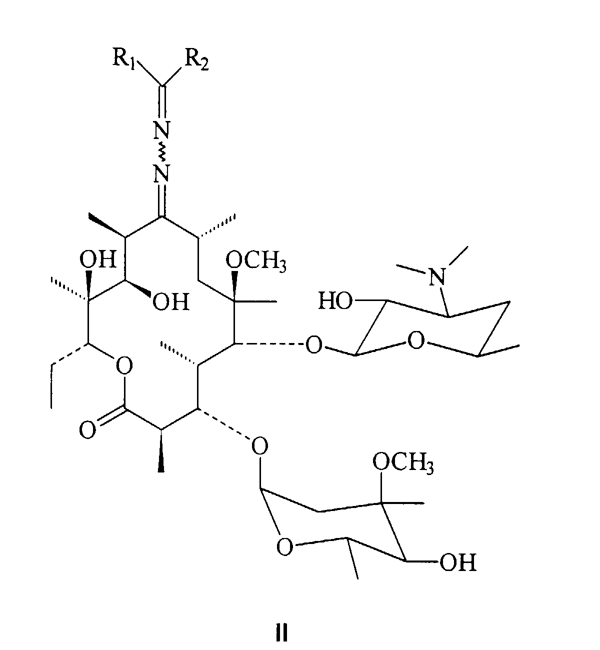 wherein R₁ and R₂ are as defined above.

Among the many erythromycin derivatives that have been described in the literature is erythromycin A 9-hydrazone prepared by reaction of erythromycin A with anhydrous hydrazine in anhydrous methanol in the course of early degradation studies on erythromycin [M.V. Sigal, Jr. et al, J. Amer. Chem. Soc. 78, 388-395 (1956)].

Erythromycin A 9-hydrazone and its N'-isopropylidene derivative have also been mentioned as intermediates in the preparation of erythromycylamine by catalytic hydrogenation [Ger. Offen. 1943781 and 1966310 (I.M. Maas et al, Lilly), E.H. Massey et al. Tetrahedron Letters, 157-160 (1970)].

The reaction of erythromycin A or B 9-hydrazone with an aldehyde or ketone has been described in US Patent No. 3,780,020 (D. Evans, Lilly) to give various antimicrobially active erythromycin 9-azine derivatives of the formula: 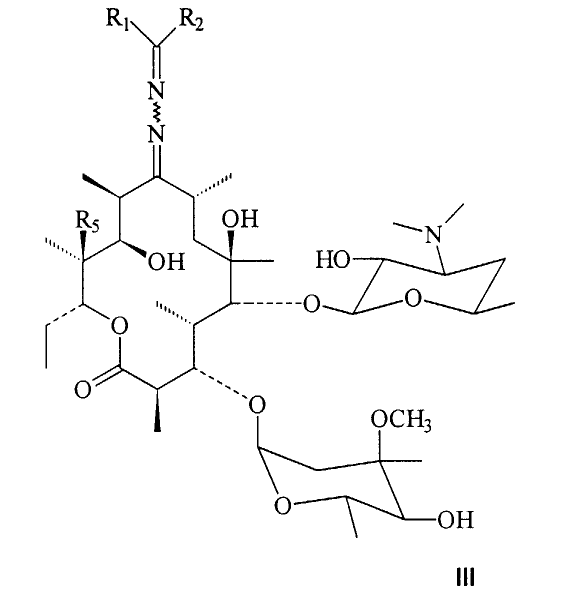 in which R₅ is H, OH; and R₁ and R₂ variously denote hydrogen, alkyl, aryl, heterocyclyl, etc. as more particularly defined in the reference.

Classically, hydrazones are prepared by the reaction of a hydrazine with a carbonyl compound, but the 9-keto group of erythromycin reacts only sluggishly with hydrazine itself and does not react with substituted hydrazines such as phenylhydrazine and semicarbazides (M.V. Sigal, Jr. op. cit.).

The present inventors have found that erythromycin A 9-hydrazone is obtained in a good yield by reacting erythromycin A with hydrazine hydrate in the presence of an acid.

Examples of acids which can be used are organic acids such as formic acid, acetic acid and propionic acid, or inorganic acids such as hydrochloric acid. The reaction solvents are alcohols, and preferably methanol. The reaction temperature may be chosen from room temperature to 62°C, and preferably from 55°C to 62°C.

An amount of hydrazine hydrate is higher than 1.0 equivalent, and preferably from 1.5 to 3.0 molar equivalents relative to erythromycin A. The acid is added in an amount to adjust the pH of the reaction solution from 6.0 to 7.0. The amount of reaction solvent is preferably from 0.9 to 5 ml, and more preferably from 1 to 3 ml to 1 g of erythromycin A.

After completion of the reaction, an aqueous basic solution, e.g. sodium hydroxide, potassium hydroxide, sodium carbonate, potassium carbonate, ammonia and the like, is added to the reaction mixture. The mixture is cooled. The precipitate is collected by filtration, washed with water and dried to give erythromycin A 9-hydrazone. The conclusion of the reaction can be recognized by determining the disappearance of erythromycin A using thin layer chromatography or high performance liquid chromatography.

For the purpose of the present invention, the erythromycin A 9-hydrazone may exist in either of two isomers and in a mixture thereof.

To protect the amino group at the 9-hydrazone position, erythromycin A 9-hydrazone is reacted with a compound represented by the general formula: 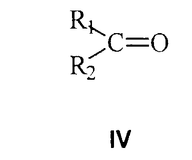 wherein R₁ and R₂ are as defined in formula I. The reaction may be similar to those usually employed for preparing the classic azines. For example, erythromycin A 9-hydrazone is reacted with a compound of formula IV in a suitable solvent to produce a compound of the formula III (wherein R₁ and R₂ are as defined in formula III and R₅ is a hydroxy group). In this reaction, the amount of the compound of formula IV is from 1 to 10 equivalents, preferably from 1 to 2 equivalents relative to erythromycin A 9-hydrazone.

The inventors used as examples the following compound having formula IV: acetone, methyl isopropyl ketone, cyclohexanone, benzaldehyde and α-tetralone. The invention is not limited though only to these compounds but has instead a general scope for compounds having general formula IV.

Examples of the solvent used for the reaction of erythromycin A 9-hydrazone with the compound of formula IV are dichloromethane, chloroform, tetrahydrofuran, methanol, ethanol, isopropanol, N,N-dimethylformamide, dimethyl sulfoxide, acetonitrile, toluene and the like. The reaction temperature ranges between the room temperature and the reflux temperature of the solvent.

In order to protect the hydroxy groups at the 2'- and 4"-positions of an erythromycin A 9-azine derivative of the formula III (wherein R₁ and R₂ are as defined in formula I and R₅ is a hydroxy group), this compound is reacted with a silylating agent. The reaction is carried out in a solvent at 0°C to the reflux temperature of the solvent, preferably at room temperature. Examples of the silylating agent used are chlorosilanes such as trimethylchlorosilane, dimethylisopropylchlorosilane and the like; silylamines such as 1,1,1,3,3,3-hexamethyldisilazane, N,N-dimethylaminotrimethylsilane, trimethylsilylimidazole and the like; silylamides such as bis(trimethylsilyl)urea, bis(trimethylsilyl)acetamide and the like or a mixture thereof. When using one of the chlorosilanes only, it is preferable to add a base. The amount of the silylating agent used is from 1 to 10 equivalents, preferably from 1 to 5 molar equivalents relative to the compound of formula III.

Examples of the solvent used for the reaction are dichloromethane, chloroform, N,N-dimethylfomamide, dimethyl sulfoxide, tetrahydrofuran, dioxane, 1,2-dimethoxyethane and the like.

Examples of the base are inorganic bases such as sodium hydroxide, potassium hydroxide, sodium carbonate, potassium carbonate and the like; and organic bases such as trimethylamine, triethylamine, tri-n-butylamine, tribenzylamine, N,N-dimethylaniline, pyridine and the like.

The silylamines can be preferably used together with the chlorosilanes or together with ammonium chloride or pyridine hydrochloride.

Alternatively, the compound of formula V can be obtained by one pot synthesis from erythromycin A 9-hydrazone. More specifically, erythromycin A 9-hydrazone is reacted with the compound of formula IV under the same conditions as described above, and then in this reaction mixture the resulting compound is reacted with the silylated agent under the same silylation conditions as described above to give a compound of formula V.:

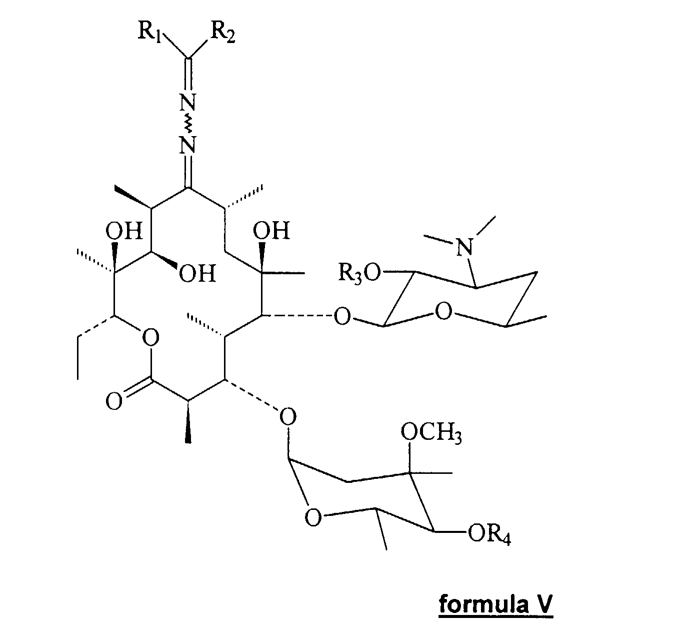

The conclusion of the reaction can be determined by observing the disappearance of erythromycin A 9-hydrazone and/or erythromycin A 9-azine derivative using thin layer chromatography or high performance liquid chromatography.

The selective methylation of the 6-hydroxy group can be carried out by the reaction of the compound of formula V with the methylating agent in a solvent in the presence of a base at -15°C to room temperature, preferably at 0° to 8°C. Examples of the methylating agent are methyl chloride, methyl bromide, methyl iodide, dimethyl sulfate, methyl p-toluenesulfonate, methyl methanesulfonate and the like. Although from 1.0 to 3.0 molar equivalents of the methylating agent can be used per mole of the compound of formula V, it is usually sufficient to use from 1.0 to 2.0 molar equivalents of it. Examples of the solvents used are polar aprotic solvents such as dimethyl sulfoxide, N,N- dimethylformamide, tetramethylene sulfoxide, hexamethylphosphoric triamide, a mixture consisting of one of these solvents and tetrahydrofuran, 1,2-dimethoxyethane, dioxane, ethyl acetate, dichloromethane and the like. Most preferred solvents are mixtures of dimethylsulfoxide and tetrahydrofuran (e.g., 1:1 or 4:3). Examples of the base are sodium hydroxide, potassium hydroxide, sodium hydride, potassium tert-butoxide, potassium hydride and the like. The amount of the base used is usually from 1 to 1.9 molar equivalents relative to the compound of formula I.

The presence of the O-methyl group at the 6-position of the 6-O-methylerythromycin A 9-azine derivative can be detected by the characteristic peak in the NMR spectrum.

Erythromycin A 9-azines may exist in either two (for symmetric carbonyl precursors) or four (for asymmetric carbonyl precursors) forms. Either isomer or their mixtures are suitable for the process.

The methylation of the hydroxy group at the 6-position of the present invention has very high selectivity.

Furthermore, the substituted silyl groups used for the protection of the hydroxy groups at the 2'- and 4"- positions can be easily removed to give the compound of formula II.

Therefore, the present invention can provide 6-O-methylerythromycin A in high yield and economically. Namely, the compound of formula I can be led to 6-O-methylerythromycin A, for example, by the following method.

The removal of the substituted silyl group (R₃ and R₄) at the 2'- and 4"-position of the compound of formula I can be carried out easily by treatment with an acid (e.g. formic acid) in an aqueous alcohol (methanol, ethanol and the like) at room temperature, and then in this reaction mixture the resulting compound of formula II is reacted with hydroxylamine to give 6-O-methylerythromycin A 9-oxime. To obtain 6-O-methylerythromycin A 9-oxime in good yield, the amount of hydroxylamine is more than 6 molar equivalents relative to the compound of formula II. The pH of the reaction mixture is from 5.5 to 8.0, and preferably from 6.0 to 7.0. The amount of reaction solvent is preferably from 0.9 to 5 ml, and more preferably from 1 to 3 ml to 1 g of the compound of formula I. The reaction temperature may be chosen from room temperature to the reflux temperature of the solvent, and preferably from 55° to 62°C. Hydroxyl amine can be added to the reaction mixture in the form of an aqueous solution, hydroxylamine hydrochloride or hydroxylamine sulfate. The conclusion of the reaction can be determined by observing the disappearance of the compound of formula I and the compound of formula II using thin layer chromatography or high performance liquid chromatography.

6-O-methylerythromycin A 9-oxime thus obtained can be easily converted into 6-O-methylerythromycin A by reaction with a deoximating agent (sodium hydrogen sulfite, titanium trichloride ammonium acetate, sodium nitrite-hydrochloric acid, sodium hydrosulfite and the like).

Thus, in another aspect of the presention invention there is provided a process for the selective methylation of a hydroxy group at the 6-position of an erythromycin A derivative comprising of converting erythromycin A to erythromycin A 9-hydrazone, converting the hydrazone to an erythromycin A 9-azine derivative of the formula III, wherein R₁ and R₂ are as defined in the formula I and R5 is a hydroxy group, protecting the hydroxyl groups at the 2'-and 4"- positions by silylation to give a compound of the formula V 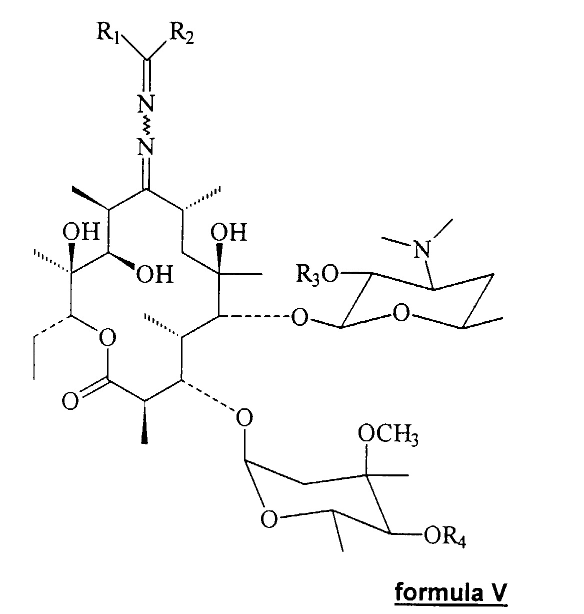 and methylating the hydroxyl group at the 6-position to obtain the compound of the formula I.

The present invention will be concretely illustrated by Examples which show the method for preparing the compound of formula I and Comparative Examples which show the method for preparing 6-O-methylerythromycin A.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example 1

### Preparation of Erythromycin A 9-hydrazone

To 500g of erythromycin A was added 650 ml of 2-propanol, and the mixture was stirred at 60°C for one hour. To the suspension was added 72g of hydrazine hydrate and 35.5g of 99% formic acid, and the mixture (pH 6.5) was stirred at 58-60°C overnight. After cooling to 15°C, to the mixture was added 600 ml of water and about 69g of 45% aqueous sodium hydroxide solution (to pH 11). Then, the mixture was stirred at ambient temperature for 2 hours and at 5°C for 5 hours. The crystals which formed were collected by filtration, washed with 900 ml of water and dried at 60°C to give 414 g of erythromycin A 9-hydrazone.
m.p. 138 - 141°C (recrystallized from ethyl acetate-hexanes).
[α]_{D}²⁰ -53.0° (c=1.0, CHCl₃).
¹H-NMR(CDCl₃) : δ(ppm) = 0.84[3H, t, CH₃(C-15)], 2.31[6H, s, 3'-N(CH₃)₂], 3.32[3H, s, 3"-OCH₃].
¹³C-NMR(CDCl₃) : δ(ppm) = 9.36(C-17), 10.64(C-15), 13.94(C-20), 40.20[3'-N(CH₃)₂], 49.38(3"-OCH₃), 96.56(C-1"), 103.02(C-1'), 168.00(C-9), 175.62(C-1).
Mass (FAB) : m/e = 748.5 (MH⁺).

### Example 2

### Preparation of erythromycin A 9-acetoneazine

50g of the compound, obtained in Example 1, were dissolved under reflux in 100 ml of acetone. The solution was then kept at ambient temperature for 8 hours and at 5°C for 16 hours. The colorless crystals which formed were collected by filtration and dried at 50°C to obtain 48g of erythromycin A 9-acetoneazine.
m.p. 131 - 134°C (recrystallized from acetone).
[α]_{D}²² -17.0° (c=1.0, CHCl₃).
¹H-NMR(CDCl₃) : δ(ppm) = 0.83 [3H, t, CH₃(C-15)], 1.87, 2.04[6H, d, C(CH₃)₂], 2.29[6H, s, 3'-N(CH₃)₂], 3.32(3H, s, 3"-OCH₃).
¹³C-NMR(CDCl₃) : δ(ppm) = 9.29 (C-17), 10.65(C-15), 14.29(C-20), 40.32 [3'-N(CH₃)₂], 49.47(3"-OCH₃), 96.43(C-1"), 103.01(C-1'), 163.82[-N=C(CH₃)₂], 174.94(C-1), 178.93(C-9).
Mass (FAB) : m/e = 788.44 (MH⁺).

### Example 3

### Preparation of 2',4"-O-bis(trimethylsilyl)erythromycin A 9-acetoneazine

To a solution of 20g of erythromycin A 9-acetoneazine, obtained in Example 2, in 100 ml of N,N-dimethylforamide were added 4.6g of pyridine hydrochloride and 15.0 ml of 1,1,1,3,3,3-hexamethyldisilazane under ice-cooling, and the mixture was stirred at ambient temperature overnight. After addition of water, the mixture was made basic (pH 11) with 5% aqueous sodium hydroxide solution and extracted with ethyl acetate. The extract was washed with, in turn, water and a saturated aqueous sodium chloride solution and dried over anhydrous magnesium sulfate. The solvent was evaporated under reduced pressure to give a residue as colorless foam. The residue was recrystallized from acetone-water (1/1) to give 22.7g of 2',4"-bis(trimethylsilyl)erythromycin A 9-acetoneazine.
m.p. 119 - 122°C (solvate with 1 mole of acetone).
[α]_{D}²² -37.5° (c=1.0, CHCl₃).
¹H-NMR(CDCl₃) : δ(ppm) = 0.11(9H, s, 2'-O-TMS); 0.15(9H, s, 4"-O-TMS); 0.84[3H, t, CH₃(C-15)]; 1.87, 2.03[6H, d, C(CH₃)₂]; 2.17[6H, s, (CH₃)₂ CO]; 2.23[6H, s, 3'-N(CH₃)₂]; 3.31(3H, s, 3"-OCH₃).
¹³C-NMR(CDCl₃) : δ(ppm) = 0.93(4"-O-TMS), 1.04(2'-O-TMS), 9.78(C-17), 10.74(C-15), 14.25(C-20), 41.00[3'-N(CH₃)₂], 49.76(3"-OCH₃), 96.73(C-1"), 102.68(C-1'),
163.54[-N=C(CH₃)₂], 175.55(C-1 ), 178.54(C-9), 206.80[(CH₃)₂CO].
Mass (FAB) : m/e = 932.66 (MH⁺).

### Example 4

### Preparation of 2',4"-O-bis(trimethylsilyl)-6-O-methylerythromycin A 9-acetoneazine

To a solution of 10g of 2',4"-O-bis(trimethylsilyl)erythromycin A 9-acetoneazine, obtained in Example 3, in 300 ml of a mixture of dimethyl sulfoxide and tetrahydrofuran (1:1) were added at ice-cooling 1.2 ml of methyl iodide and 1.2g of 85% potassium hydroxide powder, and the mixture was stirred at ice-cooling for 75 minutes. To the reaction solution were added 10 ml of 50% aqueous dimethylamine solution, and the mixture was stirred at ambient temperature for 1 hour. After addition of 300 ml of water, the mixture was extracted with ethyl acetate. The organic layer was washed with a saturated aqueous sodium chloride solution and dried over anhydrous magnesium sulfate. The solvent was evaporated under reduced pressure to give 9.3g of a crude 2',4"-O-bis(trimethylsilyl)-6-O-methylerythromycin A 9-acetoneazine as a colorless solid.
m.p. 191.5 - 194.0°C (recrystallized from ethanol).
[α]_{D}²² -150.0° (c=1.0, CHCl₃).
¹H-NMR(CDCl₃) : δ(ppm) = 0.09(9H, s, 2'-O-TMS); 0.14(9H, s, 4"-O-TMS);
0.84[3H, t, CH₃(C-15)]; 1.95, 2.06[6H, d, C(CH₃)₂]; 2.22[6H, s, 3'-N(CH₃)₂], 2.95(3H, s, 6-OCH₃); 3.31(3H, s, 3"-OCH₃).
¹³C-NMR(CDCl₃) : δ(ppm) = 0.88(4"-O-TMS), 1.06(2'-O-TMS), 9.71(C-17), 10.58(C-15), 14.87(C-20), 40.99[3'-N(CH₃)₂], 49.69(3"-OCH₃), 50.85(6-OCH₃), 96.17(C-1"), 102.54(C-1'), 163.51[-N=C(CH₃)₂], 175.86(C-1), 179.57(C-9).
Mass (FAB) : m/e = 946.84 (MH⁺).

### Example 5

### Preparation of 6-O-methylerythromycin A 9-acetoneazine

To a mixture of 1g of 2',4"-O-bis(trimethylsilyl)-6-O-methylerythromycin A 9-acetoneazine, obtained in Example 4, 5 ml of ethanol and 0.5 ml of water was added 0.2 ml of 99% formic acid, and the mixture was stirred at ambient temperature for 3 hours. The solution was diluted with 15 ml of water and made basic (pH 11) with 45% aqueous sodium hydroxide solution. Then, the mixture was kept at 5°C overnight. A colorless precipitate was collected by filtration, washed with water and dried at 50°C to obtain 0.8g of 6-O-methylerythromycin A 9-acetoneazine.
m.p. 132 - 135°C (recrystallized from ethanol-water)
[α]_{D}²² -152.0° (c=1.0, CHCl₃)
¹H-NMR(CDCl₃) : δ(ppm) = 0.84[3H, t, CH₃(C-15)]; 1.94, 2.06[6H, d, C(CH₃)₂];
2.30[6H, s, 3'-N(CH₃)₂]; 2.96(3H, s, 6-OCH3); 3.33(3H, s, 3"-OCH₃)
¹³C-NMR(CDCl₃) : δ(ppm) = 9.08(C-17), 10.48(C-15), 14.80(C-20), 40.20[3'-N(CH₃)₂], 49.36(3"-OCH₃), 50.88(6-OCH₃), 95.92(C-1"), 102.60(C-1'), 163.56[-N=C(CH₃)₂], 175.48(C-1), 179.42(C-9).
Mass (FAB) : m/e = 802.6 (MH⁺).

### Example 6

### Preparation of erythromycin A 9-cyclohexanoneazine

100g of erythromycin A 9-hydrazone, obtained in Example 1, were dissolved under reflux in 200 ml of ethyl acetate. To the solution was added 20 ml of cyclohexanone, and the mixture was refluxed for one hour and kept at ambient temperature for one hour and at 5°C overnight. The colorless crystals were collected by filtration, washed with hexanes and dried at 50°C to obtain 90.7g of erythromycin A 9-cyclohexanoneazine. After removing ethyl acetate from the filtrate, the residue was recrystallized from ethanol to give additionally 7.3g of the compound.
m.p. 140 - 143°C (recrystallized from toluene - hexanes mixture).
[α]_{D}²⁰ -3.9° (c= 1.0, CHCl₃)
¹H-NMR(CDCl₃) : δ(ppm) = 0.84[3H, t, CH₃(C-15)], 2.29[6H, s, 3-N(CH₃)₂], 3.32(3H, s, 3"-OCH₃).
¹³C-NMR(CDCl₃) : δ(ppm) = 9.12(C-17); 10.49(C-15); 14.13(C-20); 25.60, 26.06, 27.15, 28.20 and 35.45; 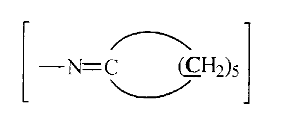 49.30(3"-OCH₃); 96.21(C-1"); 102.74(C-1'); 168.37; 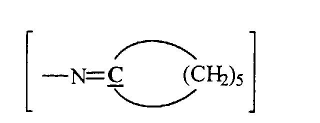 174.68(C-1); 178.42(C-9)
Mass (FAB) : m/e = 828.26 (MH⁺).

### Example 7

### Preparation of 2',4"-O-bis(trimethvlsilvl)erythromycin A 9-cyclohexanoneazine

(1) To a solution of 50g of erythromycin A 9-cyclohexanoneazine, obtained in Example 6, in 100 ml of N,N-dimethylformamide were added 6.5g of ammonium chloride and 38.0 ml of 1,1,1,3,3,3-hexamethyldisilazane under ice-cooling, and the mixture was stirred at ambient temperature overnight. Then, following a procedure similar to that of Example 3, there was obtained 57.0g of 2',4"-O-bis(trimethylsilyl)erythromycin A 9-cyclohexanoneazine as a colorless foam.
   [α]_{D}²² -34.5° (c=1.0, CHCl₃).
   ¹H-NMR(CDCl₃) : δ(ppm) = 0.08(9H, s, 2'-O-TMS); 0.11(9H, s, 4"-O-TMS); 0.81[3H, t, CH₃(C-15)]; 2.22[6H, s, 3'-N(CH₃)₂]; 3.27(3H, s, 3"-OCH₃).
   ¹³C-NMR(CDCl₃) : δ(ppm) = 0.83(4"-O-TMS); 0.96(2'-O-TMS); 9.71(C-17); 10.64(C-15); 14.18(C-20); 25.69, 26.08, 27.21, 28.28 and 35.54[-N=C(CH₂)₅]; 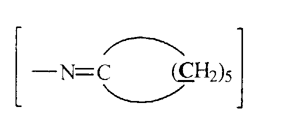 40.91[3'-N(CH₃)₂]; 49.64(3"-OCH₃), 96.61(C-1"), 102.55(C-1'), 168.00 [-N=C(CH₂)₅]; 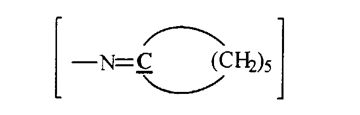 175.36(C-1 ); 178.08(C-9)
   Mass (FAB) : m/e = 972.70 (MH⁺).
(2) To a solution of 100g of erythromycin A 9-hydrazone, obtained in Example 1, in 200 ml of N,N-dimethylformamide were added 14.6 ml of cyclohexanone, and the mixture was stirred at 60°C for 4 hours. Then, 13g of ammonium chloride and 75 ml of 1,1,1,3,3,3-hexamethyldisilazane were added to the reaction mixture under ice-cooling, and the mixture was stirred at ambient temperature overnight. Then, following a procedure similar to that of Example 3, there was obtained 126g of 2',4"-O-bis(trimethylsilyl)erythromycin A 9-cyclohexanoneazine, which was identical to the compound obtained above (1).

### Example 8

### Preparation of 6-O-methylerythromycin A 9-cyclohexanoneazine

1. To a solution of 50g of 2',4"-O-bis(trimethylsilyl)erythromycin A 9-cyclohexanoneazine, obtained in Example 7, in 350 ml of a mixture of dimethyl sulfoxide and tetrahydrofuran (4:3) were added under ice-cooling 5.8 ml of methyl iodide and 5.75g of 85% potassium hydroxide powder, and the mixture was stirred at ice-cooling for 90 minutes. To the reaction solution was added 30 ml of 50% aqueous dimethylamine solution, and the mixture was stirred at ambient temperature for 1 hour. Then, the mixture was diluted with 350 ml of water and extracted with ethyl acetate. The organic layer was washed with a saturated aqueous sodium chloride solution and dried over anhydrous magnesium sulfate. The solvent was evaporated under reduced pressure to give 47.4g of 2',4"-O-bis(trimethylsilyl)-6-O-methylerythromycin A 9-cyclohexanoneazine as a foam.
2. To a solution of 20g of the compound, obtained above, in 50 ml of ethanol/ water(10/1) was added 2.8 ml of 99% formic acid, and the mixture was stirred at ambient temperature for 2 hours. Then, the reaction mixture was diluted with 60 ml of water, made basic (pH 11) with 45% aqueous sodium hydroxide solution at 15-20°C and extracted with ethyl acetate. The organic layer was washed with water and a saturated aqueous sodium chloride solution and dried over anhydrous magnesium sulfate. The solvent was evaporated under reduced pressure to give 16.2g of 6-O-methylerythromycin A 9-cyclohexanoneazine as a foam.
   m.p. 176 - 179°C (recrystallized from ethanol)
   [α]_{D}²⁵ -144.2° (c=1.0, CHCl₃)
   ¹H-NMR(CDCl₃) : δ(ppm) = 0.84[3H, t, CH₃(C-15)]; 2.29[6H, s, 3'-N(CH₃)₂]; 2.98(3H, s, 6-OCH₃); 3.33(3H, s, 3"-OCH₃).
   ¹³C-NMR(CDCl₃) : δ(ppm) = 9.07(C-17); 10.51(C-15); 14.92(C-20); 25.86, 26.16, 27.29, 28.39 and 34.85; 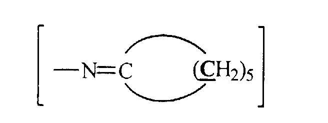 40.24[3'-N(CH₃)₂]; 49.42(3"-OCH₃); 51.07(6-OCH₃); 95.97(C-1"); 102.73(C-1"); 168.77; 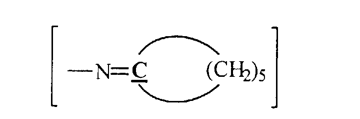 175.59(C-1), 179.71(C-9)
   Mass (FAB) : m/e = 842.24 (MH⁺).

### Example 9

### Preparation of 6-O-methylerythromycin A 9-oxime from 2',4"-O-bis(trimethylsilyl)-erythromycin A 9-cyclohexanoneazine

To a solution of 7.1g of 2',4"-O-bis(trimethylsilyl)-6-O-methylerythromycin A 9-cyclohexanoneazine, obtained above in Example 8(1), in 25 ml of methanol and 3 ml of water was added 3.0g of hydroxylamine hydrochloride, and the mixture was stirred at ambient temperature for 2 hours. Then, to the reaction mixture were added 3.5 ml of 50% aqueous hydroxylamine solution and a solution of 1g of hydroxylamine hydrochloride in 1 ml of water. The mixture (pH 6) was stirred at 60°-62°C for 24 hours. Then, the reaction mixture was cooled off to 15°C, diluted with 30 ml of water, made basic (pH 11) with 46% aqueous sodium hydroxide solution and kept at 5°C for week-end. The colorless precipitate was collected by filtration, washed with water and dried at 50°C to give 3.8g of 6-O-methylerythromycin A 9-oxime.
m.p. 248°-251°C (melted at 169°-171°C, resolidified at 180°-185°C, and melted again at 248°-251°C) (recrystallized from ethanol - petroleum ether).
[α]_{D}²² -91.3° (c=1.0, CHCl₃).
¹H-NMR(CDCl₃) δ(ppm) = 0.83[3H, t, CH₃(C-15)], 2.36[6H, s, 3'-N(CH₃)₂], 3.10(3H, s, 6-OCH₃), 3.33(3H, s, 3"-OCH₃).
¹³C-NMR(CDCl₃) : δ(ppm) = 9.21(C-17), 10.61(C-15), 14.97(C-20), 40.40[3'- N(CH₃)₂], 49.48(3"-OCH₃), 51.19(6-OCH₃), 96.03(C-1"), 102.70(C-1'), 170.36(C-9), 175.67(C-1).
Mass (FAB) : m/e = 763.4 (MH⁺).

### Example 10

### Preparation of erythromycin A 9-(3-methyl-2-butanone)azine

A mixture of 20g of erythromycin A 9-hydrazone, obtained in Example 1, and 6 ml of 3-methyl-2-butanone was heated under reflux in 20 ml of methanol for 3 hours. Then, the methanol and excess of the ketone were removed to dryness under reduced pressure to obtain 22g of erythromycin A 9-(3-methyl-2-butanone)azine as a foam.
m.p. 139-142°C (recrystallized from ethyl acetate n-hexane).
[α]_{D}²⁷ -10.0° (c=1.0, CHCl₃).
¹H-NMR(CDCl₃) : δ(ppm) = 0.84[3H, t, CH₃(C-15)], 2.34[6H, s, 3'-N(CH₃)₂], 3.32(3H, s, 3"-OCH₃).
¹³C-NMR(CDCl₃) : δ(ppm) = 9.36(C-17), 10.65(C-15), 14.43(C-20), 40.36[3'- N(CH₃)₂], 49.45(3"-OCH₃), 96.35(C-1"), 102.75(C-1'), 170.05[- N=C(CH₃)CH(CH₃)₂], 174.72(C-1), 177.48(C-9)
Mass (FAB) : m/e = 816.6 (MH⁺).

### Example 11

### Preparation of 2',4"-O-bis(trimethylsilyl)erythromycin A 9-(3-methyl-2-butanone)azine

To a solution of 8.3g of erythromycin A 9-(3-methyl-2-butanone)azine, obtained in Example 10, in 20 ml of N,N-dimethylformamide were added 1.1g of ammonium chloride and 6.4 ml of 1,1,1,3,3,3-hexamethyldisilazane under ice-cooling, and the mixture was stirred at ambient temperature overnight. Then, the reaction mixture was diluted with water, made basic (pH 11) with 5% aqueous sodium hydroxide solution and extracted with ethyl acetate. Organic layer was washed with water and a saturated aqueous sodium chloride solution and dried over anhydrous magnesium sulfate. The solvent was evaporated under reduced pressure to give 9.6g of 2',4"-O-bis(trimethylsilyl)erythromycin A 9-(3-methyl-2-butanone)azine as a foam.
[α]_{D}²⁷ -39.8° (c=1.0, CHCl₃).
¹H-NMR(CDCl₃) : δ(ppm) = 0.10(9H, s, 2'-O-TMS); 0.13(9H, s, 4"-O-TMS); 0.84[3H, t, CH₃(C-15)]; 2.23[6H, s, 3'-N(CH₃)₂]; 3.29(3H, s, 3"-OCH₃).
¹³C-NMR(CDCl₃) : δ(ppm) = 0.84(4"-O-TMS), 0.97(2'-O-TMS), 9.67(C-17), 10.63(C-15), 14.29(C-20), 40.70[3'-N(CH₃)₂], 49.68(3"-OCH₃), 96.64(C-1"), 102.48(C-1'), 169.69[-N=C(CH₃) CH(CH₃)₂], 175.44(C-1), 177.16(C-9).
Mass (FAB) : m/e = 960.5 (MH⁺).

### Example 12

### Preparation of 6-O-methylerythromycin A 9-(3-methyl-2-butanone)azine

1. To a solution of 8.0g of 2',4"-O-bis(trimethylsilyl)erythromycin A 9-(3-methyl-2-butanone)azine, obtained in Example 11, in 56 ml of a mixture of dimethyl sulfoxide and tetrahydrofurane (4:3) were added under ice-cooling 0.93ml of methyl iodide and 0.93 g of 85% KOH powder. The reaction mixture was stirred at ice-cooling for 80 minutes. Then, to the reaction solution was added 20 ml of 50% aqueous dimethylamine solution, and the mixture was stirred at ambient temperature for 1 hour. The mixture was diluted with 60 ml of water and was extracted with ethyl acetate. The organic layer was washed with a saturated aqueous sodium chloride solution and dried over anhydrous magnesium sulfate. The solvent was evaporated under reduced pressure to give 8.0g of 2',4"-O- bis(trimethylsilyl)-6-O-methylerythromycin A 9-(3-methyl-2-butanone)azine as a foam.
2. To a solution of 7.0g of the compound, obtained above, in 70 ml of ethanol / water (1/1) mixture was added 2.8 ml of 99% formic acid, and the mixture was stirred at ambient temperature for 1 hour. Then, the reaction solution was diluted with 70 ml of water, made basic (pH 11) with 5% aqueous sodium hydroxide solution and extracted with ethyl acetate. The organic layer was washed with water and a saturated aqueous sodium chloride solution and dried over anhydrous magnesium sulfate. The solvent was evaporated under reduced pressure to give 5.7g of 6-O-methylerythromycin A 9-(3-methyl-2-butanone)azine.
   m.p. 153-156°C (recrystallized from methanol).
   [α]_{D}²⁷ -127.0° (c=1.0, CHCl₃).
   ¹H-NMR(CDCl₃) : δ(ppm) = 0.83[3H, t, CH₃(C-15)]; 2.29[6H, s, 3'-N(CH₃)₂]; 3.00(3H, s, 6-OCH₃); 3.33(3H, s, 3"-OCH₃).
   ¹³C-NMR(CDCl₃) : δ(ppm) = 8.83(C-17), 10.28(C-15), 14.28(C-20), 40.01[3'-N(CH₃)₂], 49.16(3"-OCH₃), 50.73(6-OCH₃), 95.80(C-1"), 102.44(C-1'), 169.13[N=C(CH₃) CH(CH₃)₂],175.19(C-1), 177.92(C-9).
   Mass (FAB) : m/e = 830.6 (MH⁺).

### Example 13

### Preparation of erythromycin A 9-α-tetraloneazine

A mixture of 20g of erythromycin A 9-hydrazone, obtained in Example 1, and 3.55 ml of α-tetralone [3,4-dihydro-1(2H)-naphthalenone] was heated under reflux in 60 ml of toluene with a removal of water (Dean-Stark trap) for 16 hours. Then, the toluene was removed to dryness under reduced pressure to obtain 23g of erythromycin A 9-α-tetraloneazine as a foam.
m.p. 134 -137°C [recrystallized from methanol/water (1/1)].
[α]_{D}²⁷ 0.0°C (c=1.0, CHCl₃).
¹H-NMR(CDCl₃) : δ(ppm) = 0.85[3H, t, CH₃(C-15)], 2.32[6H, s, 3'-N(CH₃)₂], 3.32(3H, s, 3"-OCH₃), 7.15-8.16(4H, m, Ar).
¹³C-NMR(CDCl₃) : δ(ppm) = 9.30(C-17); 10.58(C-15); 14.48(C-20); 21.81, 27.30 and 28.87 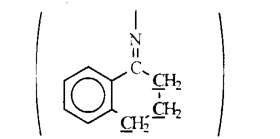 40.24[3'-N(CH₃)₂]; 49.41(3"-OCH₃); 96.40(C-1"); 102.87(C-1'); 125.36, 126.38, 128.69, 129.94, 131.98 and 140.93(Ar); 160.74 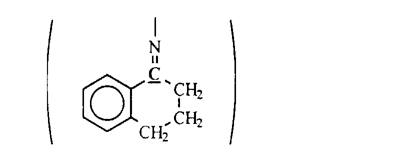 174.78(C-1); 178.18(C-9).
Mass (FAB) : m/e = 876.6 (MH⁺).

### Example 14

### Preparation of 2',4"-O-bis(trimethylsilyl)erythromycin A 9-α-tetraloneazine

To a solution of 9.8g of erythromycin A 9-α-tetraloneazine, obtained in Example 13, in 20 ml of N,N-dimethylformamide were added 1.3 g of ammonium chloride and 7.5 ml of 1,1,1,3,3,3-hexamethyldisilazane under ice-cooling, and the mixture was stirred at ambient temperature overnight. Then, following a procedure similar to that of Example 3, there was obtained 11.2g of 2',4"-O-bis(trimethylsilyl)erythromycin A 9-α-tetraloneazine as foam.
[α]_{D}²⁷ -20.2° (c=1.0, CHCl₃).
¹H-NMR(CDCl₃) : δ(ppm) = 0.13(9H, s, 2'-O-TMS), 0.15(9H, s, 4"-O-TMS), 0.86[3H, t, CH₃(C-15)], 2.26[6H, s, 3'-N(CH₃)₂], 3.31(3H, s, 3"-OCH₃), 7.15, 8.18(4H, m, Ar).
¹³C-NMR(CDCl₃) : δ(ppm) = 0.84(4"-O-TMS); 0.98(2'-O-TMS); 9.67(C-17); 10.65(C-15); 14.41(C-20); 21.86 and 27.27 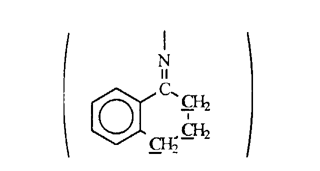 40.91 [3'-N(CH₃)₂]; 49.69(3"-OCH₃); 96.65(C-1"); 102.50(C-1'); 125.46, 126.32, 128.62, 129.84, 132.10 and 140.89 (Ar);
160.66 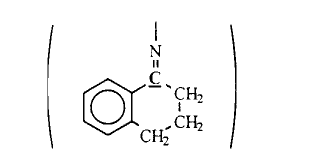 175.38(C-1); 178.10(C-9)
Mass (FAB) : m/e = 1020.6 (MH⁺).

### Example 15

### Preparation of 6-O-methylerythromycin A 9-α-tetraloneazine

1. To a solution of 9.0g of 2',4"-O-bis(trimethylsilyl)erythromycin A 9-α-tetraloneazine, obtained in Example 14, in 63 ml of a mixture of dimethyl sulfoxide and tetrahydrofuran (4:3), were added under ice-cooling 0.99 ml of methyl iodide and 0.99g of 85% potassium hydroxide powder, and the mixture was stirred at ice-cooling for 80 minutes. To the reaction mixture was added 20 ml of 50% aqueous dimethylamine solution, and the mixture was stirred at ambient temperature for 1 hour. Then, the mixture was diluted with 63 ml of water and extracted with ethyl acetate. The organic layer was washed with water and a saturated aqueous sodium chloride solution and dried over anhydrous magnesium sulfate. The ethyl acetate was evaporated under reduced pressure to give 8.6g of 2',4"-O-bis(trimethylsilyl)-6-O-methylerythromycin A 9-α- tetraloneazine as a foam.
2. To a solution of 7.0g of the compound, obtained above, in 20 ml of ethanol / water (10/1) was added 1.0 ml of 99% formic acid, and the mixture was stirred at ambient temperature for 1.5 hours. Then, the reaction mixture was diluted with 20 ml of water, made basic (pH 11) with 5% aqueous sodium hydrochloride solution and extracted with ethyl acetate. The organic layer was washed with water and a saturated aqueous sodium chloride solution and dried over anhydrous magnesium sulfate. The solvent was evaporated under reduced pressure to give 5.3g of 6-O-methylerythromycin A 9-α-tetraloneazine as a foam.
   m.p. 159 -162°C (recrystallized from methanol)
   [α]_{D}²⁷ -124.2° (c=1.0, CHCl₃).
   ¹H-NMR(CDCl₃) : δ (ppm) = 0.85[3H, t, CH₃(C-15)], 2.30[6H, s, 3'-N(CH₃)₂], 2.97(3H, s, 6-OCH₃), 3.32(3H, s, 3"-OCH₃), 7.15-8.30(4H, m, Ar).
   ¹³C-NMR(CDCl₃) : δ(ppm) = 9.10(C-17); 10.54(C-15); 15.07(C-20); 22.03, 27.30 and 28.75 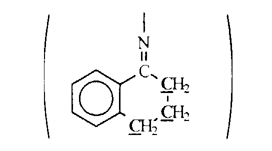 40.25[3'-N(CH₃)₂]; 49.42(3"-OCH₃); 51 .10(6-OCH₃); 95.96(C-1"); 102.61(C-1'); 125.34, 126.32, 128.63, 129.63, 132.97 and 141.04 (Ar); 160.78 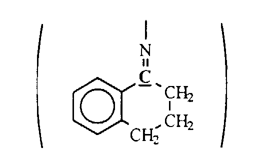 175.56(C-1 ); 179.86(C-9)
   Mass (FAB) : m/e = 890.6 (MH⁺).

### Example 16

### Preparation of erythromycin A 9-benzalazine

A mixture of 20g of erythromycin A 9-hydrazone, obtained in Example 1, and 2.73 ml of benzaldehyde was heated under reflux in 40 ml of ethyl acetate for 3 hours. Then, the solution was kept at 5°C overnight. The colorless crystals which formed were collected by filtration, washed with-ethyl acetate and dried at 50°C to give 18.1 g of erythromycin A 9-benzalazine.
m.p. 142-145°C (recrystallized from ethyl acetate).
[α]_{D}²⁸ -42.0° (c=1.0, CHCl₃).
¹H-NMR(CDCl₃). δ(ppm) = 0.85[3H, t, CH₃(C-15)], 2.31[6H, s, 3'-N(CH₃)₂], 3.31(3H, s, 3"-OCH₃), 7.42-7.78(5H, m, Ph), 8.31(1H, s, PhCH=N-).
¹³C-NMR(CDCl₃) : δ(ppm) = 9.12(C-17); 10.53(C-15); 14.25(C-20); 40.15[3'N(CH3)2]; 49.35(3"-OCH₃); 96.23(C-1"); 102.90(C-1'); 128.26, 128.66, 130.96 and 133.66(Ph); 159.66(PhCH=N-); 175.01(C-1); 184.26(C-9).
Mass (FAB) : m/e = 836.4 (MH⁺).

### Example 17

### Preparation of 2',4"-O-bis(trimethylsilyl)erythromycin A 9-benzalazine

To a solution of 10g of erythromycin A 9-benzalazine, obtained in Example 16, in 20 ml of N,N-dimethylformamide were added under ice-cooling 1.3g of ammonium chloride and 7.7 ml of 1,1,1,3,3,3-hexamethyldisilazane, and the mixture was stirred at ambient temperature overnight. Then, following a procedure similar to that of Example 3, there was obtained 11.4g of 2',4"-O-bis(trimethylsilyl)erythromycin A 9-benzalazine as foam.
[α]_{D}²⁷ -51.5° (c=1.0, CHCl₃).
¹H-NMR(CDCl₃) : δ(ppm) = 0.11(9H, s, 2'-O-TMS), 0.12(9H, s, 4"-O-TMS), 0.85[3H, t, CH₃(C-15)], 2.26[6H, s, 3'-N(CH₃)₂), 3.28(3H, s, 3"-OCH₃), 7.40 7.78(5H, m, Ph), 8.30(1H, s, PhCH=N-).
¹³C-NMR(CDCl₃) : δ(ppm) = 0.88(4"-O-TMS); 1.05(2'-O-TMS); 9.66(C-17); 10.79(C-15); 14.23(C-20); 40.96[3'-N(CH₃)₂]; 49.70(3"-OCH₃); 96.48(C-1"); 102.55(C-1'); 128.42, 128.70, 130.97 and 134.09(Ph); 159.89(PhCH=N-); 175.74(C-1); 184.12(C-9).
Mass (FAB) : m/e = 980.6 (MH⁺).

### Example 18

### Preparation of 6-O-methylerythromycin A 9-benzalazine

1. To a solution of 8.0g of 2',4"-O-bis(trimethylsilyl)erythromycin A 9-benzalazine, obtained in Example 17, in 56 ml of a mixture of dimethyl sulfoxide and tetrahydrofurane (4:3), were added under ice-cooling 0.92 ml of methyl iodide and 0.92g of 85% potassium hydroxide powder. The reaction mixture was stirred at ice-cooling for 80 minutes. Then, to the reaction solution was added 15 ml of 50% aqueous dimethylamine solution, and the mixture was stirred at ambient temperature for 1 hour. The mixture was diluted with 60 ml of water and extracted with ethyl acetate. The organic layer was washed with a saturated aqueous sodium chloride solution and dried over anhydrous magnesium sulfate. The solvent was evaporated under reduced pressure to give 7.7g of 2',4"-O-bis(trimethylsilyl)-6-O-methylerythromycin A 9-benzalazine as foam.
2. To a solution of 7.0g of the compound, obtained above, in 70 ml of ethanol / water (1:1) mixture was added 2.8 ml of 99% formic acid, and the mixture was stirred at ambient temperature for 1 hour. Then, the reaction solution was diluted with 70 ml of water, made basic (pH 11) with 5% aqueous sodium hydroxide solution and extracted with ethyl acetate. The organic layer was washed with water and a saturated aqueous sodium chloride solution and dried over anhydrous magnesium sulfate. The solvent was evaporated under reduced pressure to give 5.8g of a crude product as a foam. 5g of the crude product were purified by silica gel column chromatography (eluent, CH₂Cl₂ : CH₃OH : NH₄OH = 30:1:0.1-10:1:0.1) to give 3.83g of 6-O-methylerythromycin A 9-benzalazine.
   m.p. 142-145°C (recrystallized from chloroform-n-hexane).
   [α]_{D}²⁷ -106.5° (c=1.0, CHCl₃).
   ¹H-NMR(CDCl₃) : δ(ppm) = 0.85[3H, t, CH₃(C-15)], 2.37[6H, s, 3'-N(CH₃)₂]; 2.92(3H, s, 6-OCH₃); 3.32(3H, s, 3"-OCH₃); 7.41-7.80(5H, m, Ph); 8.36(1H, s, PhCH=N-).
   ¹³C-NMR(CDCl₃) : δ(ppm) = 9.14(C-17); 10.56(C-15); 14.66(C-20); 40.27[3'- N(CH₃)₂]; 49.37(3"-OCH₃); 51.16(6-OCH₃); 95.96(C-1"); 102.52(C-1'); 128.16, 128.71, 130.68 and 134.52(Ph); 159.14(PhCH=N-); 175.35(C-1); 184.18(C-9).
   Mass (FAB) : m/e = 850.5 (MH⁺).

### Reference Example

### Preparation of 6-O-methylerythromycin A from 6-O-methylervthromycin A 9-oxime

To a solution of 2g of 6-O-methylerythromycin A 9-oxime, obtained in Example 9, and 1.1g of sodium metabisulfite in 20 ml of ethanol / water (1/1) was added 0.25 ml of 99% formic acid, and the mixture was refluxed for 100 minutes. The reaction mixture was diluted with 30 ml of water, made basic (pH 11) with 5% aqueous sodium hydroxide solution and stirred under ice-cooling for 2 hours. The precipitate which formed was collected by filtration and recrystallized from ethanol to give 1.45g of 6-O-methylerythromycin A.
m.p. 222-225°C
[α]_{D}²² -93.2° (c=1.0, CHCl₃).

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. Novel erythromycin A derivatives of the general formula I: 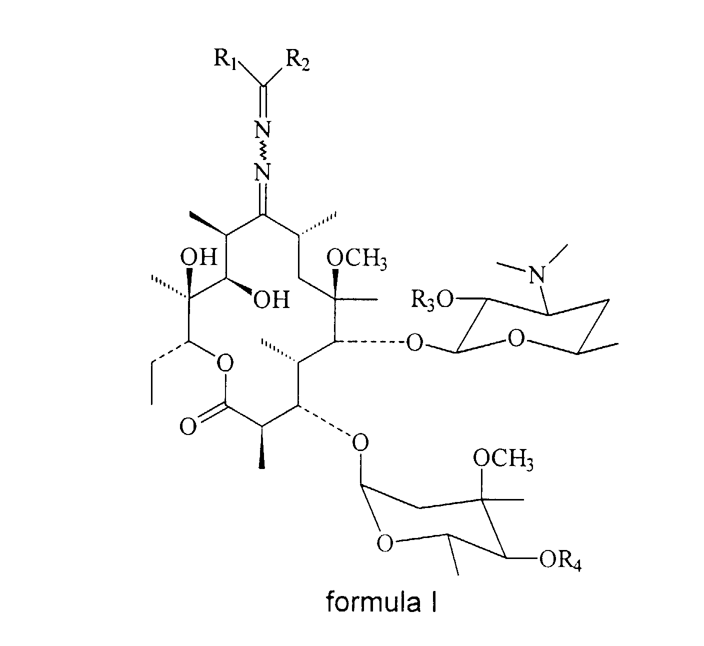 wherein R₁ is a hydrogen atom or an alkyl group having 1 to 6 carbon atoms; R₂ is an alkyl group having 1 to 6 carbon atoms or an unsubstituted or substituted aryl group; or R₁ and R₂ together form a linear or branched chain alkylene ring having 3 to 10 carbon atoms, an arylenealkylene group in which the alkylene moiety has 2 to 3 carbon atoms, or an arylenedialkylene group in which the alkylene moities have 1 to 2 carbon atoms; R₃ is a substituted silyl group; and R₄ is a hydrogen atom or substituted silyl group.

2. A compound having formula II, wherein R₁ and R₂ are as defined above

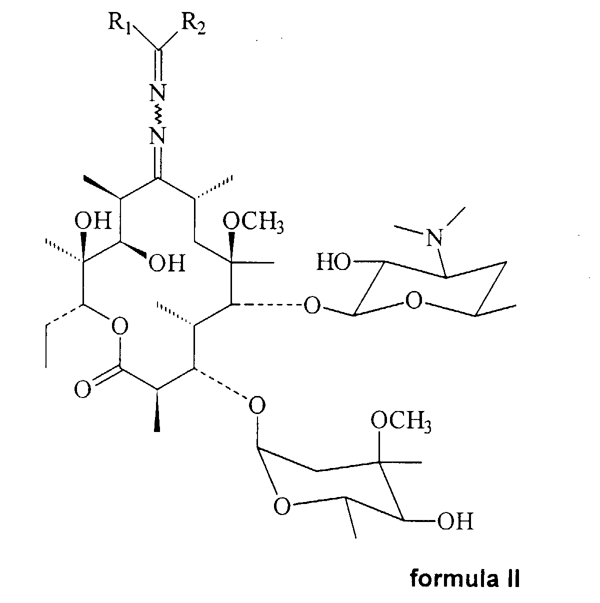

3. A compound according to claim 1 selected from the group consisting of:
(I) 6-O-methylerythromycin A 9-acetoneazine;
(II) 6-O-methylerythromycin A 9-cyclohexanoneazine;
(III) 6-O-methylerythromycin A 9-methyl-2-butanoneazine;
(IV) 6-O-methylerythromycin A 9-α-tetraloneazine;
(V) 6-O-methylerythromycin A 9-benzalazine.

4. A method for the selective methylation of the hydroxy group at the 6-position of erythromycin A comprising:
a) Converting erythromycin A to erythromycin A 9-hydrazone;
b) Converting the hydrazone to an erythromycin A 9-azine derivative of the formula III 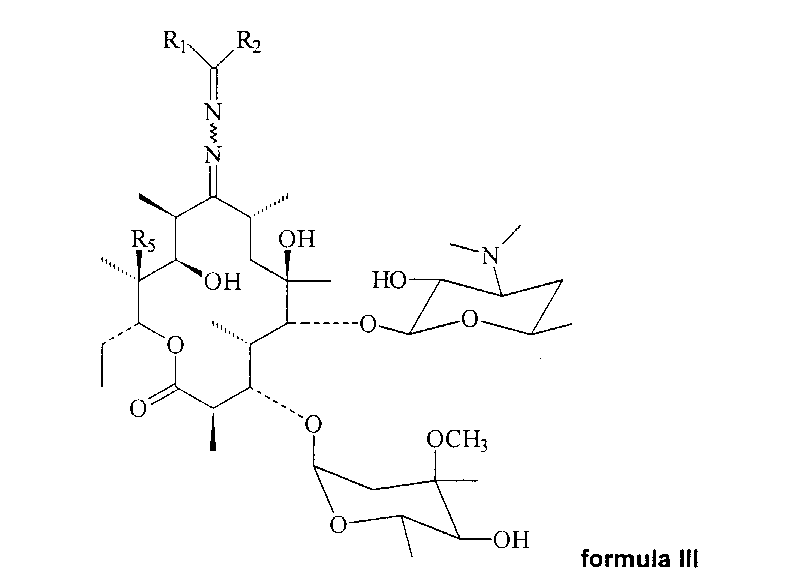 wherein R₁ and R₂ are as defined in formula I and claim 1 and R₅ is a hydroxy group; ;
c) Protecting the hydroxyl groups at the 2'- and 4"- positions by silylation to give a compound of the formula V 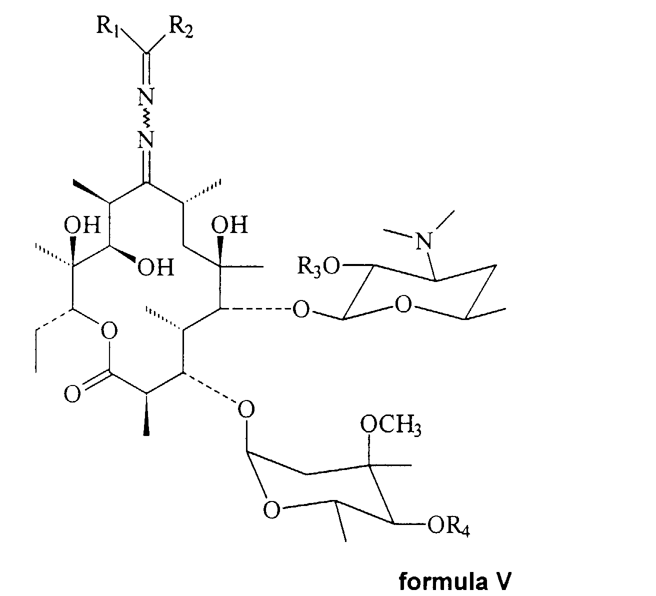 ; and
d) Methylating the hydroxyl group at the 6-position to obtain the compound of the formula I.

5. A method according to claim 4, wherein the methylation is carried out in a polar aprotic solvent in the presence of the base.

6. A method according to claim 5, wherein the base is potassium hydroxide, sodium hydroxide, sodium hydride or potassium hydride and 1.0 to 1.9 molar equivalents of the base are used per mole of the erythromycin A 9-azine derivative.

7. A method according to claim 5, wherein the polar aprotic solvent is dimethyl sulfoxide.

8. A method according to claim 5 and 7, wherein the polar aprotic solvent is a mixture of dimethyl sulfoxide and tetrahydrofuran.

9. A method according to claim 4, wherein the methylating agent is methyl iodide, methyl bromide, methyl chloride, dimethyl sulfate, methyl p-toluenesulfonate or methyl methanesufonate.

10. A method according to claim 4, wherein the amount of methylating agent is 1 to 2 molar equivalents relative to the erythromycin 9-azine derivative.

11. A method according to claim 4, wherein the methylation is conducted at 0°C to room temperature.

12. A method for the preparation of 6-O-methylerythromycin A from compounds of formula I comprising:
a) Removing the silyl protecting group with acid to obtain 6-O-methylerythromycin A 9-azine;
b) Reacting the azine with hydroxlamine to obtain 6-O-methylerythromycin A 9-oxime; and
c) Converting the 6-O-methylerythromycin A 9-oxime thus obtained to 6-O-methylerythromycin A.
